# EUROPEAN PATENT APPLICATION

(11) **EP 4 353 268 A1**
(43) Date of publication of application: **17.04.2024**
(21) Application number: 22820607.4
(22) Date of filing: 10.06.2022
(51) Int. Cl.: A61K 48/00, A61K 47/64, A61K 47/54, A61K 47/69, A61K 31/7088, A61K 31/713, A61P 35/00, A61P 29/00, A61K 9/51

(54) **NANOPARTICLE COMPRISING PEPTIDE-LIPID CONJUGATE FOR DELIVERING OLIGONUCLEOTIDE INTO TARGET CELL AND PHARMACEUTICAL COMPOSITION COMPRISING SAME**

(30) Priority: 11.06.2021 KR 20210075838
(71) Applicant: Nibec Co., Ltd., Chungcheongbuk-do 27816 (KR); Seoul National University R&DB Foundation, Seoul 08826 (KR)
(72) Inventor: PARK, Yoon Jeong, Seoul 08291 (KR); CHUNG, Chong-Pyoung, Seoul 05618 (KR); LEE, Jue-Yeon, Gwacheon-si, Gyeonggi-do 13831 (KR)
(74) Representative: Mewburn Ellis LLP
(86) International application number: PCT/KR2022/008229
(87) International publication number: WO 2022/260480

(57) **Abstract**

The present invention relates to a carrier for delivering oligonucleotides into cells for therapeutic use. To inhibit a target protein or promote expression of a target protein in a cell, a peptide-lipid conjugate is prepared, and a nanoparticle consisting of a peptide-lipid conjugate comprising oligonucleotides and a pharmaceutical composition comprising same are provided. It was confirmed that, by using the nanoparticle consisting of a peptide-lipid conjugate according to the present invention, oligonucleotides were effectively delivered into cells. In addition, by confirming that expression of a cancer-causing protein is reduced by the oligonucleotides and an anticancer effect is exhibited in an animal model in which cancer occurs, the nanoparticle consisting of a peptide-lipid conjugate was found to be effective in the delivery of oligonucleotides.

## Description

### Technical Field

The present invention relates to nanoparticles comprising peptide-lipid conjugates loaded with oligonucleotides for suppressing target proteins or promoting expression of target proteins in cells, and pharmaceutical compositions comprising the same.

### Background Art

Oligonucleotides are developed, especially, in the form of siRNAs and mRNAs, as therapeutic agents for controlling genes that cause cancer and infectious diseases. Since RNA vaccines that contain genes encoding the spike protein of the COVID 19 virus injected in the form of mRNAs to induce immune responses have recently been approved by the FDA, the application of mRNA has attracted a great deal of attention. mRNA is non-toxic because it is artificially synthesized from mRNA in the body. However, siRNAs and mRNAs are rapidly degraded *in vivo* by nucleases, are negatively charged, and cannot target specific lesions. In addition, although siRNAs and mRNAs can exert the effects thereof only when they acts in the cytoplasm, they cannot permeate the cell membrane by themselves due to the negative charge and large size thereof. Therefore, in order to overcome these limitations, carriers capable of stably delivering oligonucleotides into cells are required.

The most widely used carriers are lipid nanoparticles, and oligonucleotide carriers using various cationic lipids are known (US 2006/0083780, US 2006/0240554, US 2008/0020058, US2009/0263407, US 2009/0285881, and PCT Patent Laid-open NOS. WO 2009/086558, WO 2009/127060, WO 2009/132131, WO 2010/042877, WO 2010/054384, WO 2010/054401, WO 2010/054405, WO 2010/054406 and WO 2010/105209). Conventional cationic lipids such as CLinDMA and DLinDMA have been used for oligonucleotide delivery to the liver, but are known to have non-optimal delivery efficiencies as well as hepatotoxicity at high doses.

Lipid nanoparticles are prepared using cationic lipids, cholesterol, and polyethylene glycol (PEG). Cationic lipids allow oligonucleotides to pass through cell membranes, cholesterol allows oligonucleotides to retain the shape thereof, and PEG allows for long circulation of lipid nanoparticles. However, PEG is reported to generate antibodies against PEG, thereby inducing an anaphylactic reaction. Therefore, there is a need for novel oligonucleotide carriers capable of safely and efficiently delivering oligonucleotides into cells.

Therefore, the present inventors produced peptide-lipid conjugates using peptides comprising sequences for recognizing the target cell surface, and sequences having cell-penetrating function, promoting escape from endosomes, and binding to oligonucleotides in order to produce nanoparticles comprising peptide-lipid conjugates capable of safely and effectively delivering oligonucleotides into cells, and found that, when the nanoparticles comprising the peptide-lipid conjugates were used, oligonucleotides into the cells were effectively delivered, the expression of cancer-causing proteins was reduced by the loaded oligonucleotides, and anti-cancer effect was obtained in a cancer-induced animal model. Based thereon, the present invention was completed.

The information disclosed in this Background section is provided only for enhancement of understanding of the background of the present invention, and therefore it may not include information that forms the prior art that is already obvious to those skilled in the art.

### Summary of the Invention

It is one object of the present invention to provide a peptide-lipid conjugate capable of safely and effectively delivering oligonucleotides into cells.

It is another object of the present invention to provide nanoparticles for delivering oligonucleotides into cells, the nanoparticles in which the peptide-lipid conjugate and oligonucleotide bound to each other.

It is still another object of the present invention to provide a pharmaceutical composition comprising the nanoparticles.

It is yet another object of the present invention to provide a method for preventing or treating a disease comprising administering the nanoparticles to a subject.

It is still yet another object of the present invention to provide the use of the nanoparticles for the prevention or treatment of diseases.

It is even yet another object of the present invention to provide the use of the nanoparticles in the manufacture of a medicament for preventing or treating diseases.

In order to accomplish the above objects, the present invention provides a peptide-lipid conjugate having a peptide-lipid conjugate of the following Formula 1 as a basic unit:

[Formula 1] A-B-C-D-E

wherein A is a fatty acid or cationic lipid having 12 to 20 carbon atoms;
B is a peptide promoting endosomal escape;
C is an RNA-binding peptide;
D is a peptide having a cell-penetrating function; and
E is a peptide recognizing a target cell surface.

In addition, the present invention provides nanoparticles in which the peptide-lipid conjugate and oligonucleotide bound to each other.

In addition, the present invention provides a composition for delivering oligonucleotides into cells comprising the nanoparticles in which the peptide-lipid conjugate and oligonucleotide bound to each other.

In addition, the present invention provides a pharmaceutical composition comprising the nanoparticles in which the peptide-lipid conjugate and oligonucleotide bound to each other.

In addition, the present invention provides a method for preventing or treating diseases comprising administering the nanoparticles to a subject.

In addition, the present invention provides the use of the nanoparticles for the prevention or treatment of diseases.

In addition, the present invention provides the use of the nanoparticles in the manufacture of a medicament for preventing or treating diseases.

### Brief Description of Drawings

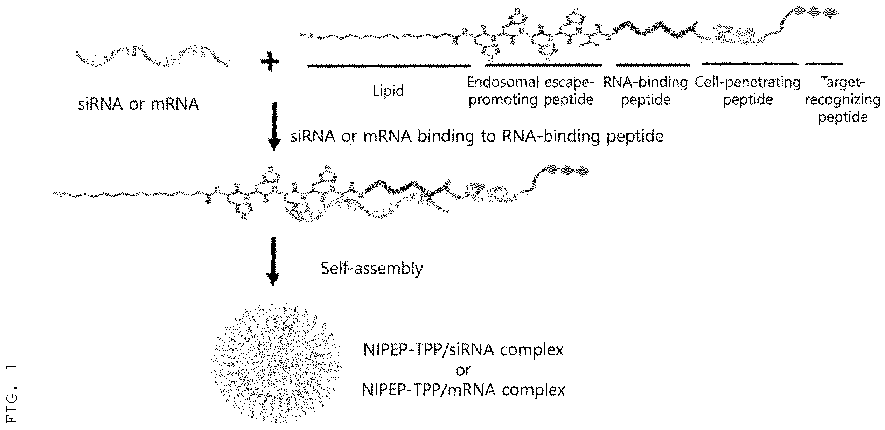

FIG. 1 is a schematic diagram illustrating a process of forming nanoparticles by self-assembly after oligonucleotides are bound to RNA-binding peptides of peptide-lipid conjugates.

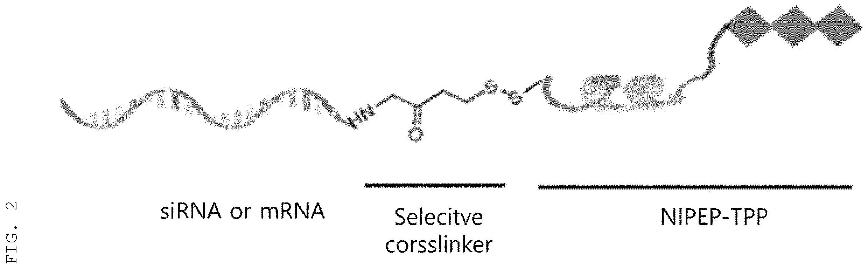

FIG. 2 is a schematic diagram illustrating a configuration in which peptides having the functions of recognizing target cell surfaces and cell-penetration are bound to mRNA using a chemical cross-linking agent.
FIG. 3A shows the results of electrophoresis of nanoparticles comprising mRNA and peptide-lipid conjugates on agarose gels at respective reaction ratios, nanoparticles formed by TEM, and the size distribution of nanoparticles. FIG. 3B shows the results of electrophoresis of nanoparticles comprising siRNA and peptide-lipid conjugates on agarose gels at respective reaction ratios, nanoparticles formed by TEM, and the size distribution of nanoparticles.
FIG. 4A shows the result of observation of the degree of penetration into cells when the cells were treated with nanoparticles comprising mRNA alone, and a combination of mRNA and peptide-lipid conjugates, and FIG. 4B shows the result of observation of the degree of penetration into cells when the cells were treated with nanoparticles comprising siRNA alone, and a combination of siRNA and peptide-lipid conjugates.
FIG. 5 is a graph showing the results of Western blot to detect the expression levels of proteins in cells by nanoparticles comprising mRNA and peptide-lipid conjugates.
FIG. 6 shows the result of Western blot, showing reduction of active KRAS and reduction of sub-signal pERK by the protein expressed in the mRNA after treating the cells with the nanoparticles comprising mRNA and peptide-lipid conjugates.
FIG. 7 is a graph showing the amount of KRAS mRNA in cells changed by nanoparticles comprising KRAS siRNA and peptide-lipid conjugates.
FIG. 8 shows the results to detect the anticancer effect of nanoparticles comprising mRNA and peptide-lipid complexes in xenograft models with lung cancer induced by H358.

### Detailed Description and Preferred Embodiments of the Invention

Unless defined otherwise, all technical and scientific terms used herein have the same meanings as appreciated by those skilled in the field to which the present invention pertains. In general, the nomenclature used herein is well-known in the art and is ordinarily used.

In the present invention, nanoparticles comprising peptide-lipid conjugates capable of safely and efficiently delivering functional oligonucleotides into cells were produced. First, peptide-lipid conjugates are produced using peptides comprising sequences for recognizing the target cell surface, and sequences having cell-penetrating function, promoting escape from endosomes, and binding to oligonucleotides and nanoparticles are produced by binding oligonucleotides to the peptide-lipid conjugates. It was found that, when the nanoparticles comprising the peptide-lipid conjugates were used, oligonucleotides into the cells were effectively delivered, the expression of cancer-causing proteins was reduced by the loaded oligonucleotides, and anti-cancer effect was obtained in a cancer-induced animal model.

Accordingly, in one aspect, the present invention is directed to a peptide-lipid conjugate having a peptide-lipid conjugate of the following Formula 1 as a basic unit:

[Formula 1] A-B-C-D-E

wherein A is a fatty acid or cationic lipid having 12 to 20 carbon atoms;
B is a peptide promoting endosomal escape;
C is an RNA-binding peptide;
D is a peptide having a cell-penetrating function; and
E is a peptide recognizing a target cell surface.

In the present invention,

A is a lipid and is, more specifically, a saturated fatty acid, an unsaturated fatty acid or a cationic lipid having 12 to 20 carbon atoms. The cationic lipid includes 3β-[N-(N',N'-dimethylaminoethane carbamoyl cholesterol (DC-Chol); 1,2-dioleoyl-3-trimethylammonium-propane (DOTAP); 1,2-dioleoyl-3-dimethylammonium-propane (DODAP); dimethyldioctadecylammonium bromide (DDAB); 1,2-dilauroyl-sn-glycero-3-ethylphosphocholine chloride (DL-EPC); N-[1-(2,3-dioleyloxy)propyl]-N-N-N-trimethylammonium chloride (DOTMA); N-[1-(2,3-dioleyloxy)propyl]-N-N-N-dimethylammonium chloride (DODMA); 1,2-dilauroyl-sn-glycero-3-ethylphosphocholine chloride (DOTMA); N,N-dioctadecyl-N,N-dimethylammonium chloride (DODAC); N-(1-(2,3-dioleyloxy)propyl)-N-2-(sperminecarboxamido)ethyl)-N,N-dimethylammonium trifluoroacetate (DOSPA); 1,2-dimyristyl oxypropyl-3-dimethylhydroxyethylammonium bromide (DMRIE); dioctadecyl amido glycyl spermine (DOGS); neutral lipids conjugated to cationic modifiers; and combinations thereof, but is not limited thereto. Further, a number of cationic lipids may be used in commercially available formulations, such as, Lipofectin (GIBCO), Lipofectamine (GIBCO), and Transfectam (Promega).

In the present invention, the lipid moiety may be present in a concentration ranging from 0 to about 60.0 mole percent of Formula 1, or from about 5.0 to about 50.0 mole percent of the lipid in the formulation.

In the present invention, B may comprise a peptide sequence that promotes the escape of endosomes. C may comprise a peptide sequence to which RNA binds. D may comprise a peptide sequence having a cell-penetrating function. E may comprise a peptide sequence that recognizes the target cell surface.

In the present invention, B is a peptide consisting of 4 to 12 histidines, D is a peptide selected from the group consisting of 4 to 12 arginines, 4 to 12 lysines, and 2 cysteines, or a combination thereof, but is not limited thereto.

In the present invention, C may be a peptide represented by any one amino acid sequence of SEQ ID NO: 1 to SEQ ID NO: 15, but is not limited thereto.
SEQ ID No. 1: LKKLLKLLKKLLKLAG
SEQ ID No. 2: IKKILIKIIKKLIKLAG
SEQ ID No. 3: LRRLLRLLRRLLRLAG
SEQ ID No. 4: LKKLLKLLOrnKLLDapLAG
SEQ ID No. 5: LRRLLRLLOrnRLLDapLAG
SEQ ID No. 6: LRKIIRLIOrnKLLDapLAG
SEQ ID No. 7: LKKLLKLLOrnKLLKLAG
SEQ ID No. 8: WKKLLKLLKKLLKLAG
SEQ ID No. 9: WRRLLRLLRRLLRLAG
SEQ ID No. 10: WRKLLRLLKKLLKLAG
SEQ ID No. 11: LKKLLDabLLKKLLKWAG
SEQ ID No. 12: LRRLLDabLLRRLLRWAG
SEQ ID No. 13: LKRLIDabIIKKLIKWAG
SEQ ID No. 14: LKKLLKWLOrnKLLDapLAG
SEQ ID No. 15: LRRLLRWLOrnRLLDabLAG

Orn = Ornithine, Dab = 1,4-diaminobutyric acid, Dap = 1,3-diaminopropionic acid.

In the present invention, E is a sequence that binds to the target cell surface and can be newly discovered and applied through phage display technology.

In one embodiment of the present invention, E may be a peptide represented by the amino acid sequence of SEQ ID NO: 16, but is not limited thereto.
SEQ ID NO: 16: CAIYPRH

In the present invention, A-B of Formula 1 may be linked by an amide bond and the amide bond of A-B may be based on chemical synthesis.

In the present invention, B-C-D-E of Formula 1 may be formed by a chemical synthesis or recombinant expression method.

In one embodiment of the present invention, B-C-D-E may be formed by synthesis based on solid-phase peptide synthesis and then an amide bond to A may be chemically formed.

In another aspect, the present invention is directed to nanoparticles in which the peptide-lipid conjugate and oligonucleotide which are bound to each other.

In the present invention, the oligonucleotide may be DNA, siRNA, miRNA or mRNA, and preferably has a function of inhibiting or increasing the expression of a target gene. The siRNA may have a nucleotide sequence complementary to a nucleotide sequence encoding a tumor-inducing protein or a disease-causing protein, but is not limited thereto.

In one embodiment of the present invention, mRNA of mutant KRAS was used to suppress the expression of mutant KRAS. The mRNA having a sequence encoding a protein that degrades KRAS may be mRNA having nucleotide sequences of SEQ ID NO: 17 and SEQ ID NO: 18, but is not limited thereto.
SEQ ID NO: 17: mRNA sequence 1 encoding protein that degrades KRAS 
5′ cap-5′UTR-
AUGGAAGUCCAAUUGUUGGAAUCUGGGGGUGGGCUUGUCCAACCUGGUGGUU
CACUCCGUCUGAGCUGCGCCGCUUCCGGAUUCACUUUCUCCACCUUUUCUAUGAAUUGG
GUUCGCCAGGCCCCAGGCAAGGGACUUGAGUGGGUUAGCUAUAUCAGCAGGACCAGUAA
AACCAUCUACUACGCCGACAGCGUUAAGGGAAGGUUCACUAUAUCCCGGGACAAUUCUA
AGAAUACUUUGUACUUGCAAAUGAACUCUUUGCGGGCAGAAGAUACUGCUGUCUACUAU
UGUGCACGAGGUAGGUUUUUUGAUUAUUGGGGUCAGGGGACCCUCGUAACCGUUUCCAG
UGGGGGCAGCGAGGGUAAGAGCUCCGGUUCAGGGUCAGAAUCUAAAAGCACUGGUGGCU
CCGAUAUACAAAUGACCCAGAGUCCAAGCUCUCUUUCAGCCAGCGUGGGAGACAGGGUG
 
ACCAUCACCUGUCGAGCAUCUCAAUCCAUUUCAUCAUACCUCAAUUGGUAUCAACAGAA

GCCUGGGGAAGCACCAAAGUUGCUUAUUUAUUCUGCCAGCGUACUGCAAAGUGGCGUAC

CUUCCCGCUUCUCCGGGUCUGGGAGUGGCACUGAUUUUACCCUCACAAUUAGCAGCCUG

CAACCCGAGGACUUCGCUACAUACUACUGCCAACAAAGUGUAAUGAUACCCAUGACAUU

CGGGCAAGGCACAAAAGUCGAGAUAAAGGGAAGUGGAGGAGGUGGCUCUAUGCCCCGCC

GCGCUGAGAACUGGGAUGAGGCCGAAGUUGGUGCUGAAGAGGCCGGAGUUGAAGAAUAU

GGGCCAGAAGAAGAUGGCGGUGAGGAAAGCGGGGCUGAAGAGAGCGGUCCAGAGGAGUC

UGGUCCAGAGGAACUUGGAGCUGAGGAGGAGAUGGAGGCCGGACGUCCACGUCCUGUCC

UGCGAUCAGUUAACUCAAGGGAGCCCUCACAGGUUAUCUUUUGUAAUCGAAGCCCUCGC

GUGGUUUUGCCCGUCUGGCUCAACUUCGAUGGUGAACCCCAACCAUACCCAACCCUCCC

CCCUGGUACCGGGCGGAGGAUUCAUUCCUAUCGGGGACACCUUUGGCUCUUCCGAGACG

CUGGGACUCACGAUGGCCUGUUGGUCAAUCAGACCGAACUGUUUGUGCCCAGCCUGAAC

GUAGACGGGCAGCCAAUUUUCGCAAACAUAACACUGCCCGUAUAUACAUUGAAAGAAAG

GUGUCUUCAGGUGGUACGCAGUCUGGUUAAACCAGAAAACUAUAGGCGUCUGGAUAUCG

UGCGCAGCCUCUAUGAGGACCUUGAAGACCACCCUAACGUGCAAAAGGACCUCGAGCGG

CUCACUCAGGAGCGCAUAGCUCAUCAGCGCAUGGGAGAUGAAAACCUUUACUUUCAGGG

UGGGAGCGGCGGCAGCGGCGGGAGUCAUCAUCACCAUCAUCAUCACCACUGA-3′

UTR- 3′ poly A tail
SEQ ID NO: 18: mRNA sequence 2 encoding protein that degrades KRAS 
5′ cap-5′UTR

AUGGAAGUGCAACUUCUCGAAUCUGGAGGGGGGUUGGUACAACCCGGCGGCA

GCUUGCGCUUGUCCUGUGCUGCCAGUGGGUUCACAUUUUCUACAUUCUCUAUGAACUGG
 
GUUAGACAAGCCCCUGGCAAAGGGCUGGAAUGGGUGAGCUAUAUAAGUCGCACCUCCAA
AACUAUUUACUAUGCAGAUAGUGUCAAGGGACGAUUUACUAUCAGCAGAGAUAACUCUA
AGAACACUCUGUAUCUCCAGAUGAAUUCCCUUCGAGCCGAAGACACCGCAGUAUAUUAC
UGUGCUAGAGGGCGCUUCUUCGACUACUGGGGGCAAGGCACUCUGGUAACUGUCAGUAG
CGGGGGUUCUGAGGGCAAAAGUUCAGGAUCUGGGUCCGAGUCUAAGUCAACUGGCGGGA
GCGACAUACAAAUGACUCAGAGCCCCAGUAGCCUGAGCGCCUCCGUUGGUGACAGAGUC
ACUAUUACAUGUCGGGCAUCUCAAUCUAUUUCCUCUUAUCUUAACUGGUAUCAGCAAAA
GCCAGGCGAAGCCCCCAAACUCCUGAUAUACUCCGCUAGUGUUCUUCAGAGUGGCGUUC
CUAGUCGCUUCAGCGGAUCCGGAAGUGGCACUGAUUUUACACUUACAAUUAGUUCCUUG
CAGCCUGAAGAUUUUGCCACAUACUACUGUCAGCAAUCAGUCAUGAUUCCCAUGACCUU
UGGGCAAGGGACCAAAGUUGAGAUCAAAGGCUCCGGAGGAGGAGGGUCUAUGGAAAACA
GAUGGCAAGUUAUGAUCGUUUGGCAAGUAGAUCGUAUGCGAAUACGCACAUGGAAAUCA
CUGGUAAAGCACCACAUGUAUGUGUCAGGCAAAGCACGAGGAUGGUUUUAUCGUCAUCA
CUAUGAGUCACCACAUCCCCGCAUCAGCUCUGAGGUGCACAUACCACUCGGUGAUGCUC
GUCUGGUCAUAACUACCUACUGGGGCCUCCACACUGGUGAAAGAGAUUGGCACCUCGGC
CAAGGAGUAAGUAUAGAAUGGCGCAAAAAGCGUUAUUCAACCCAAGUGGAUCCUGAGUU
GGCAGAUCAGUUGAUCCACUUGUAUUACUUUGACUGCUUCAGCGAUAGUGCUAUUCGGA
AGGCCCUCCUCGGGCACAUUGUAAGCCCACGGUGCGAGUAUCAAGCUGGUCAUAACAAG
GUUGGUAGCCUCCAGUACUUGGCUUUGGCUGCACUUAUAACACCCAAAAAGAUAAAACC
UCCACUCCCCUCAGUGACUAAACUCACUGAGGAUCGUUGGAACAAACCCCAGAAAACCA
AGGGGCACAGGGGUUCACACACCAUGAAUGGCCACGAGAAUUUGUAUUUCCAAGGGGGA
UCCGGGGGCAGUGGAGGGUCUCACCAUCAUCACCACCACCAUUGA-3'   UTR-3'
poly A tail
In one embodiment of the present invention, the expression of KRAS was inhibited using siRNA, and the siRNA may have nucleotide sequences of SEQ ID NO: 19 and SEQ ID NO: 20, but is not limited thereto.
SEQ ID NO: 19 and SEQ ID NO: 20: siRNA sequences that inhibit the expression of KRAS
SEQ ID NO: 19 (Sense): CAGCUAAUUCAGAAUCAUU
SEQ ID NO: 20 (AntiSense): AAUGAUUCUGAAUUAGCUG

In the present invention, the siRNA may be naive or chemically modified siRNA, or be chemically bound to the sulfhydryl group of cysteine in a cell-penetrating and target-recognizing peptide using siRNA substituted with a sulfhydryl group or an amine group at the 5' end.

In the present invention, the mRNA may encode a target protein, a recombinant chimeric protein, or a viral antigen in need of expression increase. The mRNA may be naive mRNA with a 3' cap and a 5' poly A tail, or may be bound to a sulfhydryl group of cysteine in a cell-penetrating and target-recognizing peptide using a chemical cross-linking agent after binding a sulfhydryl group or amine group to the 5' end.

In the present invention, after preparing Formula 1, oligonucleotides may be bonded to Formula 1, specifically, C in Formula 1, and allowed to stand for a predetermined period of time to allow nanoparticles to be formed by self-assembly. The nanoparticles may have a size of 10 to 200 nm.

In the present invention, the molecular weight ratio of the siRNA or mRNA to the peptide-lipid conjugate may be 1:1 to 1:100.

In the present invention, the siRNA and mRNA may be used as anticancer drugs, but are not limited thereto.

In still another aspect, the present invention is directed to a composition for delivering oligonucleotides into cells comprising the nanoparticles in which the peptide-lipid conjugate and oligonucleotide bound to each other.

In yet another aspect, the present invention is directed to a pharmaceutical composition comprising the nanoparticles in which the peptide-lipid conjugate and oligonucleotide bound to each other.

In the present invention, the pharmaceutical composition may be used for treating or preventing cancer or inflammatory diseases and the cancer includes squamous cell carcinoma, basal cell carcinoma, adenocarcinoma, hepatocellular carcinoma, renal cell carcinoma, bladder cancer, bowel cancer, breast cancer, cervical cancer, uterine cancer, colon cancer, esophageal cancer, head cancer, kidney cancer, liver cancer, lung cancer, ovarian cancer, pancreatic cancer, prostate cancer, gastric cancer, leukemia, benign and malignant lymphomas, especially Burkitt's lymphoma and non-Hodgkin's lymphoma, benign and malignant melanoma, myeloproliferative diseases, sarcoma including Ewing's sarcoma, angiosarcoma, Kaposi's sarcoma, liposarcoma, myoma, neuroepithelial sarcoma, synovial sarcoma, neurosarcoma, astrocytoma, oligodendrogliocytoma, encephalocytoma, glioblastoma, neuroblastoma, gangliocytoma, gangliocytoma, hydroblastoma, pineal tumors, meningioma, meningeal sarcoma, neurofibroma and schwannoma, testicular cancer, thyroid cancer, carcinosarcoma, Hoggins' disease, Wilms tumor, or teratocalcinoma, but is not limited thereto.

In the present invention, the pharmaceutical composition is used to treat or prevent asthma, autoimmune disease, rheumatoid arthritis, multiple sclerosis, ciliary disease, cleft palate, diabetes, heart disease, hypertension, inflammatory bowel disease, mental retardation, mood disorders, obesity, refractive error, infertility, Angelman syndrome, Canavan disease, chronic digestive disorders, Charcot-Marie-Tooth (CMT) disease, cystic fibrosis, Duchenne muscular dystrophy, hemochromatosis, hemophilia, Klinefelter syndrome, neurofibromatosis, phenylketonuria, autosomal dominant polycystic neoplasm (PKD1 or PKD2), Prader-Willi syndrome, sickle cell anemia, Tay-Sachs disease, Turner syndrome, HIV infection or HCV infection, but is not limited thereto.

According to the present invention, the pharmaceutical composition is prepared in any one formulation selected from the group consisting of injections, preparations for oral administration, patches, solutions, capsules, granules, tablets, powders, sprays, ointments, gels, mucosal formulations and suppositories, but is not limited thereto. These formulations can be prepared by a conventional method used for formulation in the art or by a method disclosed in Remington's Pharmaceutical Science (latest edition), Mack Publishing Company, Easton PA, and can be prepared in various formulations depending on each disease or component. However, the description is illustrative and formulations applicable to the present invention are not limited to those described above.

In the present invention, the pharmaceutical composition may further comprise an acceptable adjuvant and the adjuvant may be, for example, a carrier. A pharmaceutically acceptable carrier may be saline, sterile water, Ringer's solution, buffered saline, dextrose solution, maltodextrin solution, glycerol, ethanol, or a mixture of one or more of these components, and, if necessary, may further comprise other conventional additives such as antioxidants, buffers, or bacteriostatic agents. In addition, a diluent, a dispersant, a surfactant, a binder, and a lubricant may be additionally added to prepare an injection formulation such as an aqueous solution, suspension, or emulsion, pill, capsule, granule, or tablet. However, the description is illustrative and the adjuvant or carrier usable in the present invention is not limited to those described above.

In still yet another aspect, the present invention is directed to a method for preventing or treating a disease comprising administering the nanoparticles to a subject.

In even yet another aspect, the present invention is directed to the use of the nanoparticles for the prevention or treatment of diseases.

In even still yet another aspect, the present invention is directed to the use of the nanoparticles in the manufacture of a medicament for preventing or treating diseases.

In the present invention, the disease may be cancer, an inflammatory disease, asthma, an autoimmune disease, rheumatoid arthritis, multiple sclerosis, ciliary disease, cleft palate, diabetes, heart disease, hypertension, inflammatory bowel disease, mental retardation, mood disorders, obesity, refractive error, infertility, Angelman syndrome, Canavan disease, chronic digestive disorders, a Charcot-Marie-Tooth (CMT) disease, cystic fibrosis, Duchenne muscular dystrophy, hemochromatosis, hemophilia, Klinefelter syndrome, neurofibromatosis, phenylketonuria, autosomal dominant polycystic neoplasm (PKD1 or PKD2), Prader-Willi syndrome, sickle cell anemia, Tay-Sachs disease, Turner syndrome, HIV infection or HCV infection, but is not limited thereto.

In the present invention, the method, use, and usage comprise the nanoparticles according to the present invention, relate to a pharmaceutical composition comprising the nanoparticles, and descriptions overlapping with the above-described nanoparticles and pharmaceutical compositions are omitted.

Hereinafter, the present invention will be described in more detail with reference to examples. However, it will be obvious to those skilled in the art that these examples are provided only for illustration of the present invention, and should not be construed as limiting the scope of the present invention.

### Example 1: Synthesis of peptides and peptide-lipid conjugates

Amino acids and reagents required for synthesis were purchased from GL Biochem and Sigma-Aldrich. Peptides were synthesized from the C-terminus by the Fmoc solid-state chemical synthesis method using a peptide synthesizer. That is, peptides were synthesized using a rink resin (0.075 mmol/g, 100 to 200 mesh, 1% DVB crosslinking) to which Fmoc-(9-fluorenylmethoxycarbonyl) was bound as a blocking group, 50 mg of the rink resin was injected into the peptide synthesizer, the resin was swelled with DMF, and then a 20% piperidine/DMF solution was used to remove the Fmoc-group. A 0.5M amino acid solution (solvent: dimethylformamide, DMF), 1.0M DIPEA (solvent: dimethylformamide & n-methylpyrrolidone, DMF&NMP), and 0.5M HBTU (solvent: dimethylformamide, DMF) were added at 5, 10, and 5 equivalents in accordance with the sequence from the C-terminus and reacted under a nitrogen stream for 1 to 2 hours. After each of the deprotection and coupling steps, washing was performed twice with DMF and isopropanol. After coupling the last amino acid, deprotection was performed to remove the Fmoc-group. After removing the Fmoc protecting group from the last amino acid at the N-terminus, synthesis was performed by adding DIPEA (10 equivalents), HBTU (5 equivalents), and palmitic acid (5 equivalents) in the presence of DMF. The reaction was performed using a Kaiser test solution until a negative result was obtained. After the reaction was completed, the resin was washed with DMF and MeOH, and dried in a vacuum oven. A trifluoroacetic acid (TFA) cleavage cocktail was added at a rate of 20 ml per 1 g of resin, shaken for 3 hours, and a cocktail in which resin and peptides were dissolved was separated by filtering. After removing the filtered solution using a rotary evaporator, cold ether was added or an excess of cold ether was directly added to the TFA cocktail solution in which the peptide-lipid conjugate was dissolved, to crystallize the peptide-lipid conjugate into a solid phase and the peptide-lipid conjugate was separated by centrifugation. At this time, the TFA cocktail was completely removed by washing with ether several times and centrifugation. The peptide-lipid conjugate thus obtained was dissolved in distilled water and lyophilized. After completion of lyophilization, separation and purification were performed by high-performance liquid chromatography (Shimadzu, Japan). 0.1% TFA/H₂O and 0.092% TFA/acetonitrile were allowed to flow at a flow rate of 1 ml/min from 0% to 60% using a C₁₈ column with a diameter of 4.6 mm for 30 minutes. At this time, the wavelength of the ultraviolet detector was 220 nm. Purification was performed using a column with a diameter of 2.2 cm at a flow rate of 20 ml/min under the same conditions as the solvent and detection wavelength. The molecular weight of the purified peptide was determined by mass spectrometry.

### Example 2: Production of nanoparticles by conjugation of peptide-lipid conjugate and mRNA

mRNA (SEQ ID NO: 17 or SEQ ID NO: 18) of genes encoding proteins that degrade mutant KRAS and siRNA (SEQ ID NO: 19 and SEQ ID NO: 20) inhibiting KRAS expression were bound to the peptide-lipid conjugate prepared in Example 1, to produce nanoparticles.

For covalent bonding, siRNA substituted with a sulfhydryl group or amine group at the 5' end of mRNA was bound to the sulfhydryl group of cysteine in the cell-penetrating and target-recognizing peptide using a chemical cross-linking agent and nanoparticles were produced by self-assembly.

100 µg of the peptide-lipid conjugate was dissolved in 100 µL of a mixture of 90% EtOH and 10% 10 mM sodium citrate buffer as a solvent. 100 µg of each of mRNA and siRNA was dissolved in 100 µL of 10 mM sodium citrate buffer (RNase, DNase Free & PCR certified water (Tech & Innovation, BWA-8000)). The mRNA or siRNA solution and the peptide-lipid conjugate were slowly mixed in small amounts at ratios of 1:10, 1:20, 1:50, and 1:100.

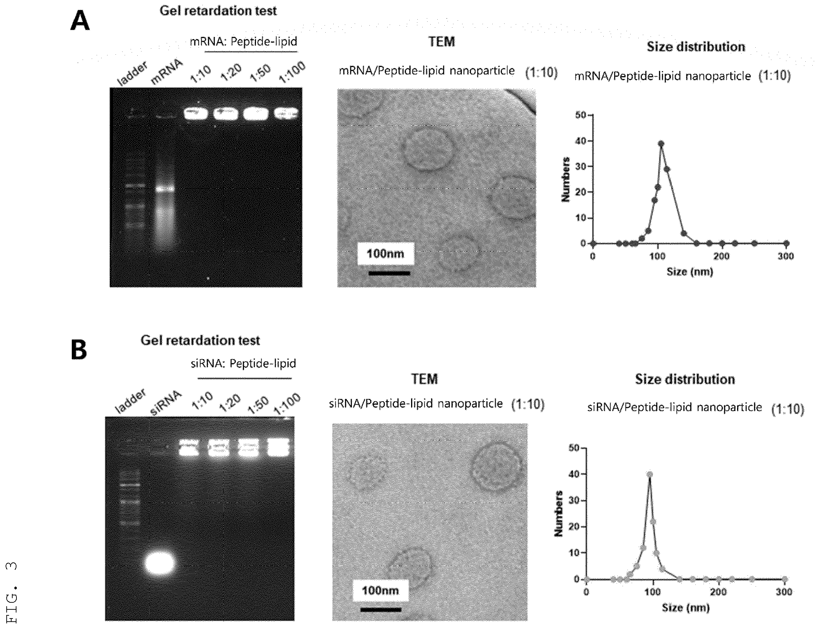

The formed nanoparticles were identified by a gel retardation test. RNA and peptide-lipid conjugates were loaded on a 2% (w/v) agarose gel in an amount of 0.1 µg of RNA at ratios of 1:10, 1:20, 1:50, and 1:100, followed by electrophoresis. It was observed that single mRNA and siRNA decreased to a positive (+) charge (FIG. 3), whereas peptide-lipid nanoparticles did not decrease from a ratio of 1:10. This means that the particle surface was changed to neutral by the peptide-lipid. In addition, nanoparticles comprising mRNA and peptide-lipid conjugates (1:10) were observed using a transmission electron microscope (TEM, JEM-1230, JEOL). Particle size was measured using dynamic light scattering (DLS, Malvern Instruments, Ltd., UK) (FIG. 3). For mRNA, there were more particles having a size of 100 nm or more, and for siRNA, there were particles having a size of about 100 nm.

### Example 3: Confirmation of delivery of nanoparticles into cells

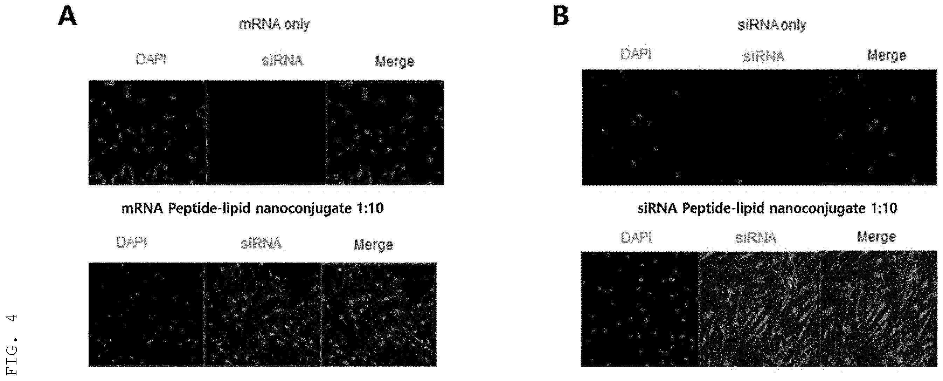

The nanoparticles produced in Example 2 were administered to the culture solution of H358 cells and it was observed that the nanoparticles were delivered into the cells. A peptide-lipid conjugate was formed using fluorescent (Cy5.5)-labeled KRAS degradation protein mRNA and fluorescent (Cy5.5)-labeled KRAS siRNA and it was observed that the nanoparticles were delivered into cells under a confocal microscope (FIG. 4).

In order to test the cell permeability of the nanoparticles containing mRNA and peptide-lipid conjugates, mRNA or siRNA was labeled with Cy5.5 (Invitrogen), and nanoparticles were produced in the same manner as in Example 2. H358 (human lung cancer cells, CRL-5807, ATCC) was seeded at a density of 1×10⁴. After 24 hours, the culture medium was treated with 100 nM nanoparticles containing Cy5.5-mRNA and peptide-lipid conjugate or containing Cy5.5-siRNA and peptide-lipid conjugate for 1 hour. Then, the cells were fixed with a 10% neutral formalin solution and measured using a confocal differential scanning microscope.

As a result, a remarkably increased amount of the nanoparticles was observed in the cytoplasm of H358 cells (FIG. 4). Therefore, it was found that the nanoparticles comprising mRNA or siRNA-loaded peptide-lipid conjugates can effectively penetrate cells.

### Example 4: Identification of expression of KARS degradation protein by mRNA delivered into cells

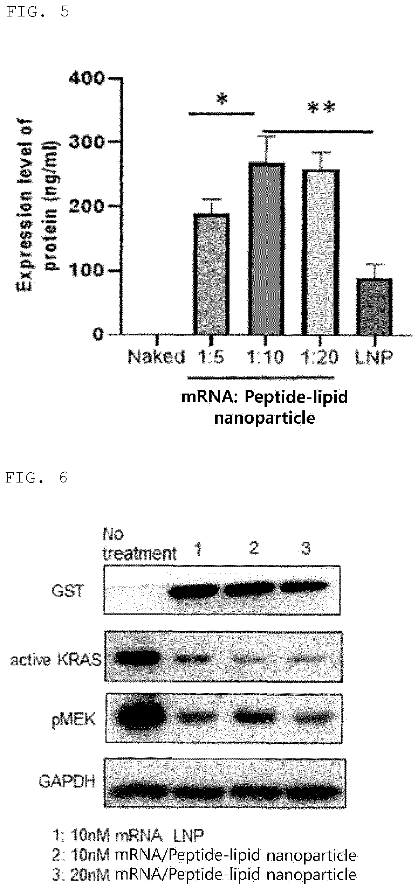

In addition, the amounts of proteins expressed in cells by nanoparticles comprising mRNA and peptide-lipid conjugates were detected by Western blotting (FIG. 5).

H358 cells were seeded at a density of 70% in a 6-well plate. After 16 hours, cell starvation was performed overnight with DMEM containing 0.5% FBS. The cells were treated with a medium containing nanoparticles comprising mRNA and peptide-lipid conjugates at concentrations of 10 nM and 20 nM for 16 hours. 10 nM mRNA mixed with lipofectamine was used as a control. The protein was lysed using RIPA lysis buffer (25 mM Tris HCl pH 7.6, 150 mM NaCl, 1% NP-40, 1% sodium deoxycholate, 0.1% SDS) containing protease and phosphatase inhibitors. The protein was quantified by BCA protein assay (23227, Thermo Scientific) and the expression levels of KRAS degraded protein and actin protein were determined by western blotting. Western blotting was performed by loading the sample in the same amount along with the size marker on an 8% SDS PAGE gel, performing electrophoresis for about 2 hours, and then transferring the same to a nitrocellulose membrane. The transferred membrane was blocked with 5% skim milk for 1 hour and reacted overnight with a primary antibody (GST, Abcam, ab19256) at a ratio of 1:1,000. Then, the membrane was washed with TBST containing 0.1% tween-20, and reacted with an HRP-linked secondary antibody (anti-Rabbit, A90-116P, BETHYL Lab.; anti-Mouse, A120-101P, BETHYL Lab.) for 1 hour, and chemiluminescence was identified with an ECL substrate.

As a result, as shown in FIG. 5, the nanoparticles comprising the peptide-lipid conjugate according to the present invention exhibited much higher amounts of KRAS degradation proteins expressed by mRNA in cells than lipid nanoparticles such as lipofectamine and the expression was the highest at the ratio of 1:10 (*p<0.5, **p<0.01).

### Example 5: Confirmation of reduction of active KARS by KRAS degradation protein mRNA delivered into cells

The result of Western blot shows reduction of active KRAS and reduction of sub-signal pERK in the cells injected with the nanoparticles comprising mRNA and peptide-lipid conjugates in Example 4 (FIG. 6).

H358 cells were seeded at a density of 70% in a 6-well plate. After 16 hours, cell starvation was performed overnight with DMEM containing 0.5% FBS. The cells were treated with a medium containing nanoparticles comprising mRNA and peptide-lipid conjugates at concentrations of 10 nM and 20 nM for 16 hours. As a control, 10 nM mRNA mixed with lipofectamine was used. The protein was lysed using RIPA lysis buffer (25 mM Tris HCl pH 7.6, 150 mM NaCl, 1% NP-40, 1% sodium deoxycholate, 0.1% SDS) containing protease and phosphatase inhibitors. The protein was assayed by BCA protein assay (23227, Thermo Scientific) and the expression levels of FER tyrosine kinase and actin protein were detected by western blotting. Western blotting was performed by loading the sample in the same amount along with the size marker on an 8% SDS PAGE gel, performing electrophoresis for about 2 hours, and then transferring the same to a nitrocellulose membrane. The transferred membrane was blocked with 5% skim milk for 1 hour and reacted overnight with a primary antibody (GST, Abcam, ab19256) at a ratio of 1:1000. Then, the membrane was washed with TBST containing 0.1% tween-20, and reacted with an HRP-linked secondary antibody (anti-Rabbit, A90-116P, BETHYL Lab.; anti-Mouse, A120-101P, BETHYL Lab.) for 1 hour, and chemiluminescence was identified with an ECL substrate.

As a result, as shown in FIG. 6, the nanoparticles comprising the peptide-lipid conjugate according to the present invention effectively reduced active KRAS and sub-signal pMEK, as compared to lipid nanoparticles such as lipofectamine.

### Example 6: Confirmation of reduction of messenger KARS by KRAS siRNA delivered into cells

H358 cells were seeded at a density of 70% in a 6-well plate. After 16 hours, cell starvation was performed overnight with DMEM containing 0.5% FBS. The cells were treated with a medium containing nanoparticles comprising KRAS siRNA and a peptide-lipid conjugate at a concentration of 100 nM for 16 hours. As a control, 100 nM of siRNA mixed with Lipofectamine was used.

Quantitative real-time PCR was performed in the following manner. For mRNA quantification, total RNA was isolated using the Qiagen RNeasy kit. cDNA was synthesized using Verso cDNA kit (Thermo Scientific) using 500 ng RNA. mRNA values were measured with a 7500 Fast Real-Time PCR System (Applied Biosystems) based on SYBR Green.

The primers used herein were as follows.
KRAS:
F-TGACCTGCTGTGTCGAGAAT (SEQ ID NO: 21),
R-TTGTGGACGAATATGATCCAA (SEQ ID NO: 22);
18S:
F-CGCCGCTAGAGGTGAAATTC (SEQ ID NO: 23),
R-TTGGCAAATGCTTTCGCTC (SEQ ID NO: 24)

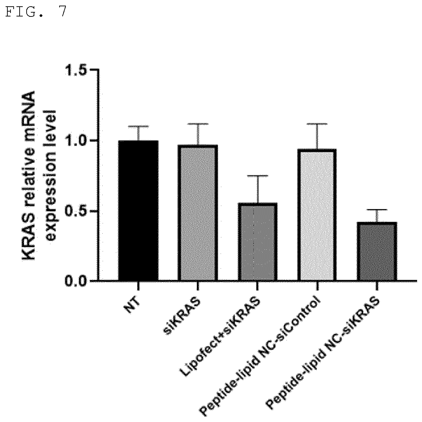

18S rRNA was used as a housekeeping gene (control gene) . PCR was performed using reverse-transcribed RNA and 100 ng/µL of sense and antisense primers (total volume 20 µL) were used. 40 cycles were performed and each cycle included denaturation at 95°C for 15 seconds, annealing for 1 minute, and extension at 60°C. The results are shown in FIG. 7. Treatment with KRAS siRNA and peptide-lipid conjugate (peptide-lipid NC) exhibited the most decreased KRAS mRNA and siRNA alone or control siRNA showed no effect.

### Example 7: Confirmation of anticancer effects in lung cancer animal models

The nanoparticles comprising mRNA and the peptide-lipid conjugate produced in Example 2 were administered to the xenograft mouse model with lung cancer induced by the H358 cell line, and the anticancer effect thereof was identified. A mixture of 100 µL of 1×10⁶ H358 cells and Matrigel (BD Bioscience, San Diego, CA, USA) was implanted on the thigh of 5-6-weeks old female Balb/c nude mouse (5-6 weeks old; Japan SLC Inc, Hamamatsu, Japan) to form a tumor with a size of 100 mm³. Nanoparticles comprising mRNA and peptide-lipid conjugates and lipid nanoparticles (LNP) prepared from mRNA and lipofectamine were each injected intraperitoneally twice a week at a dose of 1 µg/kg for 30 days. The size of the tumor was measured with a vernier caliper every 3 to 4 days, and on the 30^{th} day, the mice were sacrificed to remove the tumor and the removed tumor was imaged.

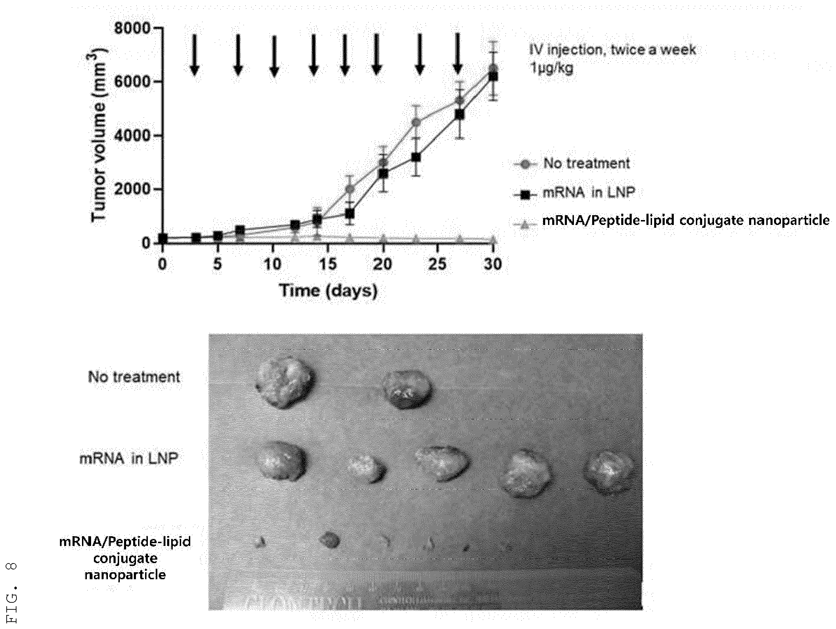

As a result, as shown in FIG. 8, the tumor size did not increase in the experimental group administered the nanoparticles comprising the peptide-lipid conjugate, and the tumor size of the lipid particles prepared with Lipofectamine continued to increase.

### Industrial Applicability

The nanoparticles consisting of peptide-lipid conjugates linked with oligonucleotides according to the present invention effectively deliver therapeutic oligonucleotides into cells and change the expression of target proteins by oligonucleotides, thus being useful as therapeutic agents.

Although specific configurations of the present invention have been described in detail, those skilled in the art will appreciate that this description is provided to set forth preferred embodiments for illustrative purposes, and should not be construed as limiting the scope of the present invention. Therefore, the substantial scope of the present invention is defined by the accompanying claims and equivalents thereto.

### Sequence List Free Text

An electronic file is attached.

## Claims

1. A peptide-lipid conjugate having a peptide-lipid conjugate of the following Formula 1 as a basic unit:
[Formula 1] A-B-C-D-E
wherein A is a fatty acid or cationic lipid having 12 to 20 carbon atoms;
B is a peptide promoting endosomal escape;
C is an RNA-binding peptide;
D is a peptide having a cell-penetrating function; and
E is a peptide recognizing a target cell surface.

2. The peptide-lipid conjugate according to claim 1, wherein B is a peptide consisting of 4 to 12 histidines.

3. The peptide-lipid conjugate according to claim 1, wherein D is a peptide selected from the group consisting of 4 to 12 arginines, 4 to 12 lysines, and 2 cysteines, or a combination thereof.

4. The peptide-lipid conjugate according to claim 1, wherein C is a peptide represented by any one amino acid sequence of SEQ ID NO: 1 to SEQ ID NO: 15.

5. The peptide-lipid conjugate according to claim 1, wherein E has an amino acid sequence represented by SEQ ID NO: 16.

6. The peptide-lipid conjugate according to claim 1, wherein A-B of the basic unit of Formula 1 is linked by an amide bond.

7. The peptide-lipid conjugate according to claim 6, wherein the amide bond of A-B is formed based on chemical synthesis.

8. The peptide-lipid conjugate according to claim 1, wherein B-C-D-E is produced by a chemical synthesis or recombinant expression method.

9. Nanoparticles in which the peptide-lipid conjugate according to any one of claims 1 to 8 and oligonucleotide bound to each other.

10. The nanoparticles according to claim 9, wherein the oligonucleotide is selected from the group consisting of DNA, siRNA, miRNA and mRNA.

11. The nanoparticles according to claim 9, wherein the oligonucleotide inhibits or increases the expression of a target gene.

12. The nanoparticles according to claim 10, wherein the siRNA has a nucleotide sequence complementary to a nucleotide sequence encoding a tumor-inducing protein or a disease-causing protein.

13. The nanoparticles according to claim 12, wherein the siRNA is naive or chemically modified siRNA, or is chemically bound to a sulfhydryl group of cysteine in cell-penetrating and target-recognizing peptides using siRNA substituted with a sulfhydryl group or an amine group at the 5' end.

14. The nanoparticles according to claim 10, wherein the mRNA encodes a target protein, a recombinant chimeric protein, or a viral antigen in need of expression increase.

15. The nanoparticles according to claim 9, wherein the nanoparticles are formed through self-assembly of the peptide-lipid conjugate after binding the oligonucleotide to C in Formula 1.

16. The nanoparticles according to claim 15, wherein the nanoparticles have a size of 10 to 200 nm.

17. The nanoparticles according to claim 10, wherein the oligonucleotide is siRNA or mRNA, and a molecular weight ratio of the siRNA or mRNA to the peptide-lipid conjugate is 1:1 to 1:100.

18. A composition for delivering oligonucleotides into cells comprising the nanoparticles in which the peptide-lipid conjugate according to claim 9 and oligonucleotide bound to each other.

19. A pharmaceutical composition comprising the nanoparticles in which the peptide-lipid conjugate according to claim 9 and oligonucleotide bound to each other.

20. The pharmaceutical composition according to claim 19, in which used to treat or prevent cancer or inflammatory diseases.

21. The pharmaceutical composition according to claim 20, wherein the cancer is selected from the group consisting of squamous cell carcinoma, basal cell carcinoma, adenocarcinoma, hepatocellular carcinoma, renal cell carcinoma, bladder cancer, bowel cancer, breast cancer, cervical cancer, uterine cancer, colon cancer, esophageal cancer, head cancer, kidney cancer, liver cancer, lung cancer, ovarian cancer, pancreatic cancer, prostate cancer, gastric cancer, leukemia, benign and malignant lymphomas, benign and malignant melanoma, myeloproliferative diseases, sarcoma including Ewing's sarcoma, angiosarcoma, Kaposi's sarcoma, liposarcoma, myoma, neuroepithelial sarcoma, synovial sarcoma, neurosarcoma, astrocytoma, oligodendrogliocytoma, encephalocytoma, glioblastoma, neuroblastoma, gangliocytoma, gangliocytoma, hydroblastoma, pineal tumors, meningioma, meningeal sarcoma, neurofibroma and schwannoma, testicular cancer, thyroid cancer, carcinosarcoma, Hoggins' disease, Wilms tumor, and teratocalcinoma.

22. The pharmaceutical composition according to claim 20, in which used to treat or prevent a disease selected from the group consisting of asthma, autoimmune disease, rheumatoid arthritis, multiple sclerosis, ciliary disease, cleft palate, diabetes, heart disease, hypertension, inflammatory bowel disease, mental retardation, mood disorders, obesity, refractive error, infertility, Angelman syndrome, Canavan disease, chronic digestive disorders, a Charcot-Marie-Tooth (CMT) disease, cystic fibrosis, Duchenne muscular dystrophy, hemochromatosis, hemophilia, Klinefelter syndrome, neurofibromatosis, phenylketonuria, autosomal dominant polycystic neoplasm (PKD1 or PKD2), Prader-Willi syndrome, sickle cell anemia, Tay-Sachs disease, Turner syndrome, HIV infection, and HCV infection.
